# EUROPÄISCHE PATENTSCHRIFT

(11) **EP 2 506 789 B1**
(45) Veröffentlichungstag und Bekanntmachung des Hinweises auf die Patenterteilung: **09.07.2014**
(21) Anmeldenummer: 10778958.8
(22) Anmeldetag: 03.11.2010
(51) Int. Cl.: A61B 18/12

(54) **HOCHFREQUENZCHIRURGIEGENERATOR**
HIGH FREQUENCY SURGERY GENERATOR
GÉNÉRATEUR CHIRURGICAL HAUTE FRÉQUENCE

(30) Priorität: 01.12.2009 DE 102009044720; 25.01.2010 DE 102010000184
(43) Veröffentlichungstag der Anmeldung: 10.10.2012
(73) Patentinhaber: ERBE Elektromedizin GmbH, 72072 Tübingen (DE)
(72) Erfinder: SCHALL, Heiko, 72622 Nürtingen (DE); FRITZ, Martin, 72070 Tübingen (DE)
(74) Vertreter: Bohnenberger, Johannes
(86) Internationale Anmeldenummer: PCT/EP2010/066726
(87) Internationale Veröffentlichungsnummer: WO 2011/067061

(56) Entgegenhaltungen:
- WO-A1-98/27880
- DE-A1- 2 250 574
- US-A1- 2004 193 148

## Beschreibung

Die Erfindung betrifft einen Hochfrequenzchirurgiegenerator zum Erzeugen einer HF-Ausgangsspannung nach dem Oberbegriff des Patentanspruchs 1.

Die Hochfrequenzchirurgie wird heutzutage in vielen medizinischen Bereichen eingesetzt. Hierbei wird durch ein relativ hohe Spannung, die an einer monopolaren oder bipolaren Elektrode anliegt, biologisches Gewebe gezielt geschädigt oder geschnitten. Zum Erzeugen dieser Spannungen wird üblicher Weise ein HochfrequenzChirurgiegenerator (HF-Chirurgiegenerator) verwendet.

Zum Schneiden und/oder Koagulieren des biologischen Gewebes werden verschiedenartige Modulationen verwendet. Hierfür müssen z.B. Schwingungspakete (Bursts) schnell beginnen und enden.

Die EP 1 776 929 A1 beschreibt einen Hochfrequenzchirurgiegenerator, bei dem die Ausgangsspannung des HF-Chirurgiegenerators schnell reduziert werden kann. Hierbei wird bei Überschreiten einer Schwelle die Ausgangsspannung des HF-Chirurgiegenerators schnell reduziert, indem die Energie des Netzteils durch Zuschalten eines zusätzlichen Lastbauelements thermisch umgesetzt wird. Nachteilig hieran ist, dass die entstehende Wärme abgeführt werden muss, der HF-Chirurgiegenerator technisch aufwändig ist und ein schlechtes Schneidverhalten aufweist.

Die DE 102 18 895 A1 beschreibt einen weiteren Hochfrequenzchirurgiegenerator, bei dem die Ausgangsspannung des Hochfrequenzchirurgiegenerators schnell reduziert werden kann. Hierbei wird die Ausgangsspannung des Netzteils erniedrigt, indem die Energie des Ausgangskondensators in die Gleichspannungsversorgung zurückgespeist wird. Dadurch werden die Energiespeicher auf der Ausgangsseite der Gleichspannungsversorgung sowie im Leistungsoszillator schnell entladen.

Hierdurch wird die Ausgangsspannung des Hochfrequenzchirurgiegenerators schnell reduziert. Ein Nachteil hiervon ist, dass der Hochfrequenzchirurgiegenerator ein schlechtes Schneidverhalten bietet und die Schaltung kompliziert und technisch aufwändig ist.

Die US 2004/0193148 A1 beschreibt einen Hochfrequenzchirurgiegenerator, bei dem Überspannungen in der Ausgangsspannung des HF-Chirurgiegenerators schnell herunterregelt werden können. Hierbei wird, sobald die Ausgangsspannung des HF-Chirurgiegenerators oberhalb einer Grenzspannung liegt, die Resonanzfrequenz des Oszillators durch Hinzuschalten weiterer Bauteile verändert. Folglich stimmt die gleichbleibende Anregungsfrequenz nicht mehr mit der Resonanzfrequenz des Oszillators überein, wodurch die Ausgangsspannung des HF-Chirurgiegenerators unter die Grenzspannung, d.h. auf die Höhe einer Normalausgangsspannung, reduziert und Funkenbildung verhindert wird. Nachteilig hieran ist, dass die Schaltung technisch aufwändig ist und eine Funkenbildung, die zum Schneiden vorteilhaft ist, verhindert wird und somit der HF-Chirurgiegenerator ein schlechtes Schneidverhalten bietet.

Eine Vorrichtung der hier angesprochenen Art ist auch aus der DE 22 50 574 A1 bekannt. Diese Druckschrift offenbart den nächstliegenden Stand der Technik.

Der Erfindung liegt die Aufgabe zu Grunde, einen HF-Chirurgiegenerator aufzuzeigen, der technisch einfach ist und ein verbessertes Schneidverhalten aufweist.

Diese Aufgabe wird durch einen HF-Chirurgiegenerator nach Anspruch 1 und ein Verfahren nach Anspruch 3 gelöst.

Ein wesentlicher Punkt der Erfindung besteht darin, dass der HF-Chirurgiegenerator über zwei Betriebsmodi verfügt: Im ersten Betriebsmodus wird eine Ausgangsschaltung durch einen HF-Leistungsoszillator mit einer Resonanzfrequenz der Ausgangsschaltung angeregt. In einem zweiten Betriebsmodus wird die Ausgangsschaltung durch den HF-Leistungsoszillator mit einer anderen Frequenz angeregt, die nicht ihrer Resonanzfrequenz entspricht. Beim Übergang vom ersten Betriebsmodus zum zweiten Betriebsmodus wird die Ausgangsspannung des HF-Chirurgiegenerators schnell und schlagartig erniedrigt.

Hierdurch entstehen oberschwingungsbehaftete Ausgangsspannungen, wodurch ein verbessertes Schneidverhalten des HF-Chirurgiegenerator erzielt wird. Zudem ist der HF-Chirurgiegenerator technisch einfach und kostengünstig.

Der HF-Leistungsoszillator umfasst Schaltelemente, insbesondere Transistoren zur Steuerung eines Vorzeichens einer Anregungsspannung, mit denen der HF-Leistungsoszillator die Ausgangsschaltung abhängig von einem Vorzeichen eines Anregungsstroms des HF-Leistungsoszillators im ersten Betriebsmodus in einem Rückkopplungsbetrieb anregt und zur Einstellung der zweiten Anregungsfrequenz in einem zweiten, zwangsgesteuerten Betriebsmodus. Hierdurch kann im ersten Betriebsmodus die Ausgangsschaltung mit einer Resonanzfrequenz mit möglichst geringem Energieverlust technisch einfach angetrieben werden. Vorteilhaft hieran ist auch, dass im zweiten Betriebsmodus die Frequenz der Anregungsspannung technisch einfach eingestellt werden kann.

Der HF-Chirurgiegenerator umfasst ein einstellbares Frequenzsteuergerät zum Messen eines Vorzeichens des Anregungsstroms und zum Öffnen und/oder Schließen der Schaltelemente zum Steuern eines Vorzeichens der Anregungsspannung im ersten Betriebsmodus und zum Einstellen der zweiten Anregungsfrequenz im zweiten Betriebsmodus. Hierdurch kann technisch einfach das Vorzeichen des Anregungsstroms erfasst und das Vorzeichen der Anregungsspannung im ersten Betriebsmodus entsprechend eingestellt werden. Vorteilhaft hieran ist auch, dass die Anregungsfrequenz im zweiten Betriebsmodus gezielt eingestellt werden kann.

In einer Ausführungsform umfasst der Hochfrequenzchirurgiegenerator eine steuerbare Betriebsmodusumschaltvorrichtung zum Umschalten von dem ersten Betriebsmodus zum zweiten Betriebsmodus zum Erzeugen einer oberwellenbehafteten Ausgangsspannung. Ein Vorteil hiervon ist, dass gezielt eine oberwellenbehaftete Ausgangsspannung eingestellt werden kann, die zum Schneiden des Gewebes sehr vorteilhaft ist.

Bevorzugte Ausführungsformen der Erfindung ergeben sich aus den Unteransprüchen. Nachfolgend wird die Erfindung anhand von Zeichnungen von Ausführungsbeispielen näher erläutert. Hierbei zeigen
- Fig. 1: eine schematische Ansicht eines HF-Chirurgiegenerators und
- Fig. 2: eine schematische Ansicht eines Schaltungsaufbaus in einem Netzteil und in einem Leistungsoszillator eines HF-Chirurgiegenerators.

Bei der nachfolgenden Beschreibung werden für gleiche und gleich wirkende Teile dieselben Bezugsziffern verwendet.

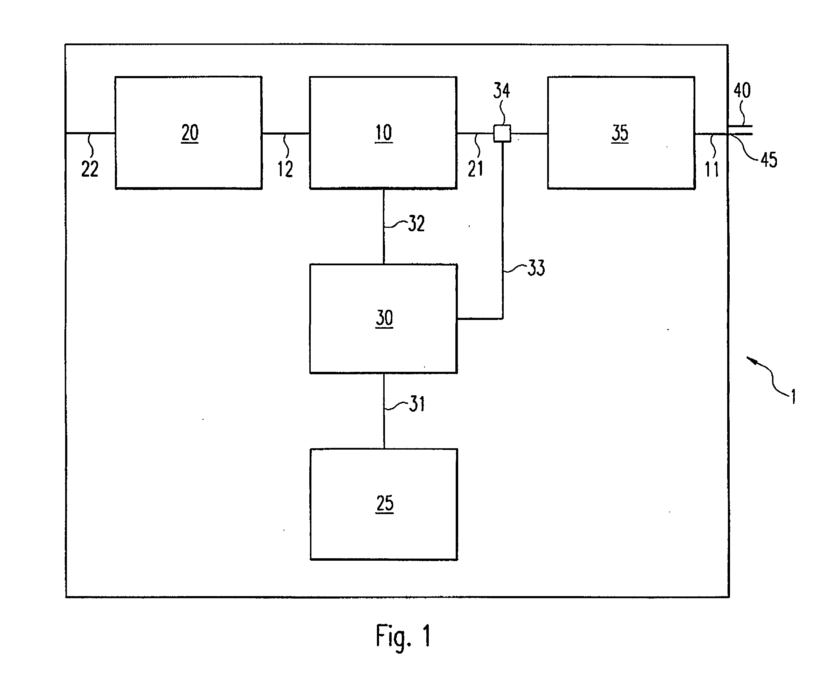

Fig. 1 zeigt eine schematische Ansicht eines HF-Chirurgiegenerators. Der HF-Chirurgiegenerator verfügt über ein Netzteil 20, das über Verbindungsleitungen 22 mit einer Strom- und Spannungsversorgung (nicht gezeigt) verbunden ist und über eine Verbindungsleitung 12 einen Leistungsoszillator 10 mit elektrischer Energie versorgt. Der Leistungsoszillator 10 ist über eine Verbindungsleitung 21 mit einer Ausgangsschaltung 35 verbunden. Der Leistungsoszillator 10 treibt die Ausgangsschaltung 35 mit einer Anregungsfrequenz an. Die Ausgangsschaltung 35 ist über eine Verbindungsleitung 11 mit einem der Ausgänge 45 des HF-Chirurgiegenerators verbunden. An den Ausgängen 45 kann eine monopolare oder bipolare Elektrode 40 angeschlossen werden, an der eine Ausgangsspannung des HF-Chirurgiegenerators anliegt.

Im ersten Betriebsmodus wird die Ausgangsschaltung 35 durch den Leistungsoszillator 10 mit einer ersten Anregungsfrequenz angeregt, die einer oder der Resonanzfrequenz der Ausgangsschaltung 35 entspricht. Bei einer Resonanzfrequenz der Ausgangsschaltung 35 ist die Amplitudenübertragungsfunktion | A(f) | = | uₐᵤₛ / uₑᵢₙ | (wobei uₑᵢₙ die Spannung ist, die am Leistungsoszillatorausgang anliegt ist, und uₐᵤₛ die Spannung ist, die am Ausgang der Ausgangsschaltung, also am Ausgang des HF-Chirurgiegenerators anliegt) an ihrem Maximum. Bei der Resonanzfrequenz wird die maximale Leistung übertragen. In der Regel ist dies auch der Arbeitspunkt mit dem höchsten energetischen Wirkungsgrad.

Diese Anregung der Ausgangsschaltung 35 im ersten Betriebsmodus mit einer Anregungsfrequenz, die seiner Resonanzfrequenz oder einer seiner Resonanzfrequenzen entspricht, wird folgendermaßen erreicht: Das Frequenzsteuergerät 30 misst über eine Verbindungsleitung 33 mit Hilfe eines Strommessgeräts 35 das Vorzeichen des Ausgangsstroms des Leistungsoszillators 10, d.h. in welcher Phase sich der Ausgangsstrom gerade befindet. Abhängig hiervon stellt das Frequenzsteuergerät 30 über eine Verbindungsleitung 32 das Vorzeichen der Ausgangsspannung des Leistungsoszillators 10 entsprechend ein, so dass die Ausgangsspannung des Leistungsoszillators 10 in Phase zum Ausgangsstrom des Leistungsoszillators 10 liegt.

Im zweiten Betriebsmodus wird die Ausgangsschaltung 35 durch den Leistungsoszillator 10 mit einer zweiten Anregungsfrequenz angeregt, die nicht der ersten Anregungsfrequenz entspricht, indem unabhängig von der Phase des Ausgangsstroms des Leistungsoszillators 10 die Ausgangsspannung des Leistungsoszillators 10 auf eine Frequenz eingestellt wird, die der zweiten Anregungsfrequenz entspricht. Die zweite Anregungsfrequenz wird durch das Frequenzsteuergerät 30 eingestellt. Die zweite Anregungsfrequenz befindet sich nicht in der Nähe der Resonanzfrequenz der Ausgangsschaltung 35, d.h. die zweite Anregungsfrequenz unterscheidet sich nicht nur unwesentlich von der Resonanzfrequenz, weil die Amplitudenübertragungsfunktion sich bei einer Frequenz nahe der Resonanzfrequenz in der Nähe ihres Maximums befindet.

Bei dieser zweiten Anregungsfrequenz befindet sich die Amplitudenübertragungsfunktion nicht an bzw. fern von ihrem Maximum. Dadurch sinkt die Ausgangsspannung bzw. -leistung der Ausgangsschaltung 35 und somit wird die Ausgangsspannung des HF-Chirurgiegenerators schnell reduziert bzw. erniedrigt. Des Weiteren erhöht sich hierdurch schlagartig der Generatorinnenwiderstand des HF-Chirurgiegenerators.

Beim Betrieb des HF-Chirurgiegenerators im zweiten Betriebsmodus liefert die Ausgangsschaltung 35 stark oberschwingungsbehaftete Ausgangsstrom- bzw. Ausgangsspannungsformen. Dies begünstigt die Funkenbildung an der Elektrode 40. Eine Funkenbildung an der Elektrode 40 ist insbesondere gewünscht, wenn biologisches Gewebe mit der Elektrode 40 geschnitten werden soll. Durch diesen zweiten Betriebsmodus wird es ermöglicht, bei einem ansonsten klirrarmen Chirurgiegenerator oberschwingungsbehaftete Ausgangsspannungen bzw. Ausgangsspannungsformen zu erzeugen.

Eine Betriebsmodusumschaltvorrichtung 25 ist über eine Verbindungsleitung 35 mit dem Frequenzsteuergerät 30 verbunden. Hierdurch kann zwischen einem ersten Betriebsmodus und einem zweiten Betriebsmodus bzw. vom ersten Betriebsmodus zum zweiten Betriebsmodus umgeschaltet werden. Gezielt kann hiermit der zweite Betriebsmodus eingestellt werden, in dem Gewebe geschnitten werden kann.

Zudem kann beispielsweise ein Not-Knopf mit der Betriebsumschaltvorrichtung 25 verbunden sein, so dass bei einem Notfall die Ausgangsspannung des HF-Chirurgiegenerators, d.h. die Spannung an der Elektrode 40, durch Betätigen des Not-Knopfes durch den Chirurgen oder eine andere Person schnell reduziert werden kann.

Der HF-Chirurgiegenerator kann eine Anzeige, beispielsweise in der Form von Anzeigelampen, umfassen, die anzeigt, in welchem Betriebsmodus sich der HF-Chirurgiegenerator gerade befindet.

Der Leistungsoszillator 10 treibt die Ausgangsschaltung 35 im zweiten Betriebsmodus stets mit der gleichen niederohmigen Quellimpedanz.

Dadurch wird gewährleistet, dass die Ausgangsfrequenz der Ausgangsschaltung 35 stets der gestellten Frequenz des Leistungsoszillators 10 entspricht.

Durch den hier aufgezeigten HF-Chirurgiegenerator wird auch bei einer aus einer leitfähigen Flüssigkeit, wie z.B. physiologische Kochsalzlösung, bestehenden Umgebung der angeschlossenen Elektrode 40 das Schneidverhalten des HF-Chirurgiegenerators aufgrund der oberschwingungsbehafteten Ausgangsspannung im zweiten Betriebsmodus verbessert.

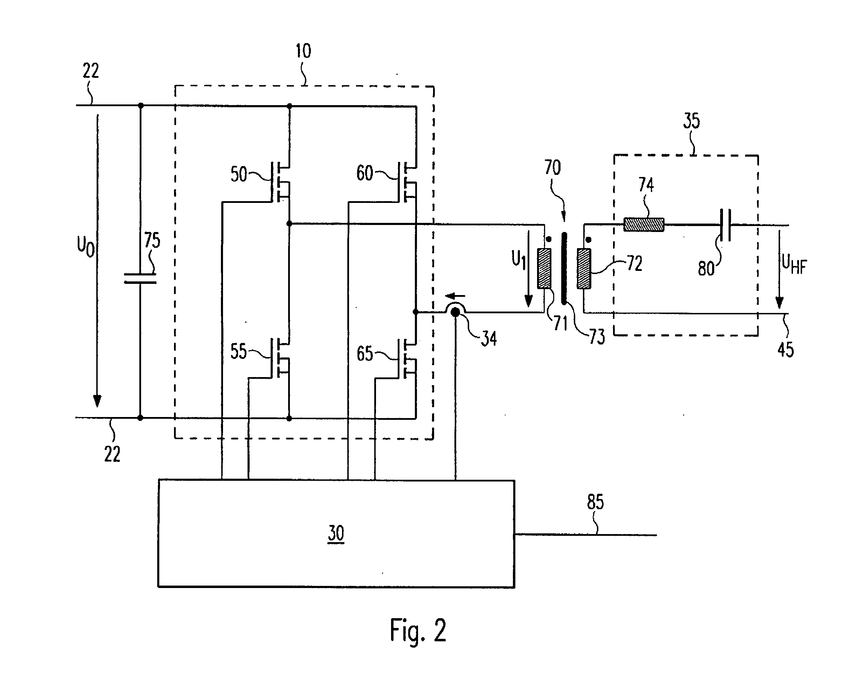

Fig. 2 zeigt einen Schaltungsaufbau einer zweiten Ausführungsform eines HF-Chirurgiegenerators. Der Leistungsoszillator 10 ist über Verbindungsleitungen 22 mit einem Netzteil (nicht gezeigt) verbunden. Das Netzteil liefert eine Spannung U0, die über eine erste Kapazität 75 anliegt. Der Leistungsoszillator 10 ist mit der Ausgangsschaltung 35 über einen Transformator 70 verbunden. Durch den Transformator 70 werden die Stromkreise des HF-Leistungsoszillators 10 und der Ausgangsschaltung 35 galvanisch voneinander getrennt. Der Transformator 70 umfasst eine erste Induktivität 71 und eine zweite Induktivität 72, die über einen Magnetkern 73 gekoppelt sind. Der Leistungsoszillator 10 umfasst vier Transistoren 50, 55, 60, 65. An dem Transformator liegt auf der Seite des Leistungsoszillators 10 eine Spannung U1 an. Ein Frequenzsteuerungsgerät 30 misst mit Hilfe eines Strommessgeräts 35 das Vorzeichen eines Stroms I1 am Ausgang des Leistungsoszillators 10.

Die Eingänge des ersten Transistors 50 und des dritten Transistors 60 sind über eine der Verbindungsleitungen 22 mit dem Netzteil verbunden. Der Ausgang des ersten Transistors 50 ist mit einem Anschluss der ersten Induktivität 71 und mit dem Eingang des zweiten Transistors 55 verbunden. Der Ausgang des dritten Transistors 60 ist mit einem Ausgang des Strommessgeräts 35 und mit dem Eingang des vierten Transistors 65 verbunden. Die Ausgänge des zweiten Transistors 55 und des vierten Transistors 65 sind über eine andere der Verbindungsleitungen 22 mit dem Netzteil verbunden.

Das Frequenzsteuerungsgerät 30 ist über eine Verbindungsleitung 85 mit einem Frequenzgeber (hier nicht gezeigt) verbunden. Das Frequenzsteuerungsgerät 30 ist jeweils mit dem Steuerungsanschluss des ersten 50, zweiten 55, dritten 60 und vierten Transistors 65 verbunden.

Das Frequenzsteuergerät 30 schaltet, wenn der vom Strommessgerät 34 gemessene Wert von I1 größer Null ist, den ersten Transistor 50 und den vierten Transistor 65 auf Durchgang. Stellt das Frequenzsteuergerät 30 fest, dass I1 kleiner gleich Null ist, so schaltet das Frequenzsteuergerät 30 den dritten Transistor 60 und den zweiten Transistor 55 auf Durchgang. Die anderen beiden Transistoren sind jeweils auf Sperrung geschaltet.

Die Ausgangsschaltung 35 umfasst des Weiteren eine dritte Induktivität 74 und eine zweite Kapazität 80. Diese sind mit einem der Ausgänge 45 des HF-Chirurgiegenerators verbunden. An den Ausgängen 45 liegt die Hochfrequenzspannung UHF an. Durch die Steuerung des Frequenzsteuergeräts 30 befinden sich die Ausgangsspannung U1 und der Ausgangsstrom I1 des HF-Leistungsoszillators in Phase und die Amplitudenübertragungsfunktion befindet sich an ihrem bzw. in der Nähe ihres Maximums.

Im zweiten Betriebsmodus wird die Ausgangsschaltung 35 mit einer resonanzfremden Anregungsfrequenz angeregt. Als resonanzfremde Frequenz ist eine Frequenz zu verstehen, die nicht gleich der Resonanzfrequenz des Ausgangskreises 35 ist und die auch nicht nur unwesentlich von der Resonanzfrequenz abweicht. Im zweiten Betriebsmodus werden unabhängig von dem Vorzeichen des Anregungsstroms I1 der erste Transistor 50 und der vierte Transistor 65 sowie der zweite Transistor 55 und der dritte Transistor 60 im Wechsel jeweils mit der halben Periodendauer der gewünschten zweiten Anregungsfrequenz auf Durchgang geschaltet. Die jeweils beiden anderen Transistoren sind auf Sperrung geschaltet. Diese zweite Anregungsfrequenz wird durch den Frequenzgeber an das Frequenzsteuergerät 30 gesandt. Die vom Frequenzgeber gesandte zweite Anregungsfrequenz ist einstellbar. Die Amplitudenübertragungsfunktion befindet sich im zweiten Betriebsmodus (weiter) entfernt von ihrem Maximum.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste:

- 1: HF-Chirurgiegenerator
- 10: HF-Leistungsoszillator
- 11: Verbindungsleitung Ausgangsschaltung-Ausgang
- 12: Verbindungsleitung Netzteil-HF-Leistungsoszillator
- 20: Netzteil
- 21: Verbindungsleitung HF-Leistungsoszillator-Ausgangsschaltung
- 22: Verbindungsleitung Netzteil-Stromquelle
- 25: Betriebsmodusumschaltvorrichtung
- 30: Frequenzsteuergerät
- 31: Verbindungsleitung Frequenzsteuergerät-Betriebsmodusumschaltvorrichtung
- 32: Verbindungsleitung Frequenzsteuergerät-HF-Leistungsoszillator
- 33: Verbindungsleitung Frequenzsteuergerät-Strommessgerät
- 34: Strommessgerät
- 35: Ausgangsschaltung
- 40: Elektrode
- 45: Ausgänge des HF-Chirurgiegenerators
- 50: erster Transistor
- 55: zweiter Transistor
- 60: dritter Transistor
- 65: vierter Transistor
- 70: Transformator
- 71: Erste Induktivität
- 72: Zweite Induktivität
- 73: Magnetkern
- 74: Dritte Induktivität
- 75: Erste Kapazität
- 80: zweite Kapazität
- 85: Verbindungsleitung Frequenzgeber-Frequenzsteuerung

## Patentansprüche

1. Hochfrequenzchirurgiegenerator zum Erzeugen einer HF-Ausgangsspannung mit einer Ausgangsschaltung (35) und einem HF-Leistungsoszillator (10) zum Anregen der Ausgangsschaltung (35) mit einer ersten Anregungsfrequenz, in der der vom HF-Leistungsoszillator zur Ausgangsschaltung fließende Anregungsstrom oszilliert und die einer Resonanzfrequenz der Ausgangsschaltung (35) entspricht, in einem ersten Betriebsmodus wobei
der HF-Leistungsoszillator (10) zum Anregen der Ausgangsschaltung (35) mit einer von der ersten Anregungsfrequenz unterschiedlichen zweiten Anregungsfrequenz, die nicht der Resonanzfrequenz der Ausgangsschaltung (35) entspricht, in einem zweiten Betriebsmodus ausgebildet ist,
**dadurch gekennzeichnet, dass**
der HF-Leistungsoszillator (10) Schaltelemente, insbesondere Transistoren (50, 55, 60, 65), zur Steuerung des Vorzeichens der Anregungsspannung umfasst, mit denen der HF-Leistungsoszlllator (10) die Ausgangsschaltung (35) abhängig vom Vorzeichen des Anregungsstroms im ersten Betriebsmodus in einem Rückkopplungsbetrieb anregt und zur Einstellung der zweiten Anregungsfrequenz in einem zweiten, zwangsgesteuerten Betriebsmodus, und dass
der Hochfrequenzchirurgiegenerator ein einstellbares Frequenzsteuergerät (30) zum Messen des Vorzeichens des Anregungsstroms und zum Öffnen und/oder Schließen der Schaltelemente zum Einstellen des Vorzeichens der Anregungsspannung im ersten Betriebmodus und zum Einstellen der zweiten Anregungsfrequenz im zweiten Betriebsmodus umfasst.

2. Hochfrequenzchirurgiegenerator nach Anspruch 1 **gekennzeichnet durch** eine steuerbare Betriebsmodusumschaltvorrichtung zum Umschalten von dem ersten Betriebsmodus zum zweiten Betriebsmodus zum Erzeugen einer oberwellenbehafteten Ausgangsspannung.

3. Verfahren zum Betreiben eines Hochfrequenzchirurgiegenerators mit einem HF-Leistungsoszillator (10) und einer Ausgangsschaltung (35), folgende Schritte umfassend:
- Anregen der Ausgangsschaltung (35) durch den HF-Leistungsoszillator (10) in einem ersten Betriebsmodus mit einer ersten Anregungsfrequenz, in der der vom HF-Leistungsoszillator zur Ausgangsschaltung fließende Anregungsstrom oszilliert und die einer Resonanzfrequenz der Ausgangsschaltung (35) entspricht, zum Erzeugen der HF-Ausgangsspannung,
- Anregen der Ausgangsschaltung (35) durch den HF-Leistungsoszillator (10) in einem zweiten Betriebsmodus mit einer von der ersten Anregungsfrequenz unterschiedlichen zweiten Anregungsfrequenz, die nicht der Resonanzfrequenz der Ausgangsschaltung (35) entspricht, zum beschleunigten Erniedrigen der HF-Ausgangsspannung,
- Messen des Vorzeichens des Anregungsstroms und
- Öffnen und/oder Schließen von Schaltelementen, insbesondere Transistoren (50, 55, 60, 65), zum Steuern des Vorzeichens der Anregungsspannung im HF-Leistungsoszillator (10) abhängig vom Vorzeichen des Anregungsstroms im ersten Betriebsmodus in einem Rückkopptungsbetrieb und zur Einstellung der zweiten Anregungsfrequenz in einem zweiten, zwangsgesteuerten Betriebsmodus.

## Claims

1. Electrosurgical generator for generating an RF output voltage, with an output circuit (35) and an RF power oscillator (10) for exciting the output circuit (35) with a first excitation frequency, in which the excitation current flowing from the RF power oscillator to the output circuit oscillates, and which corresponds to a resonant frequency of the output circuit (35), in a first operating mode,
wherein
the RF power oscillator (10) is embodied in a second operating mode for exciting the output circuit (35) with a second excitation frequency that differs from the first excitation frequency and does not correspond to the resonant frequency of the output circuit (35),
**characterized in that**
the RF power oscillator (10) comprises switching elements, more particularly transistors (50, 55, 60, 65) for controlling the sign of the excitation voltage, by means of which switching elements the RF power oscillator (10) excites the output circuit (35) in a feedback operation in the first operating mode depending on the sign of the excitation current, and for setting the second
excitation frequency in a second, forced operating mode,
and **in that**
the electrosurgical generator comprises an adjustable frequency control instrument (30) for measuring the sign of the excitation current and for opening and/or closing the switching elements for setting the sign of the excitation voltage in the first operating mode and for setting the second excitation frequency in the second operating mode.

2. Electrosurgical generator according to Claim 1, **characterized by** a controllable operating-mode switching device for switching from the first operating mode to the second operating mode for producing an output voltage with harmonics.

3. Method for operating an electrosurgical generator with an RF power oscillator (10) and an output circuit (35), comprising the following steps:
- exciting the output circuit (35) by the RF power oscillator (10) in a first operating mode with a first excitation frequency, in which the excitation current flowing from the RF power oscillator to the output circuit oscillates, and which corresponds to a resonant frequency of the output circuit (35), for producing the RF output voltage,
- exciting the output circuit (35) by the RF power oscillator (10) in a second operating mode with a second excitation frequency that differs from the first excitation frequency and does not correspond to the resonant frequency of the output circuit (35) for the accelerated reduction of the RF output voltage,
- measuring the sign of the excitation current and
- opening and/or closing switching elements, more particularly transistors (50, 55, 60, 65), for controlling the sign of the excitation voltage in the RF power oscillator (10) depending on the sign of the excitation current in a feedback operation in the first operating mode and for setting the second excitation frequency in a second, forced operating mode.

## Revendications

1. Générateur chirurgical de hautes fréquences servant à former une tension de sortie HF et présentant un circuit de sortie (35) et un oscillateur de puissance HF (10) qui excite le circuit de sortie (35) par une première fréquence d'excitation et dans lequel dans un premier mode de fonctionnement, le courant d'excitation délivré par l'oscillateur de puissance HF et s'écoulant vers le circuit de sortie est mis en oscillation, la fréquence d'excitation correspondant à une fréquence de résonance du circuit de sortie (35),
l'oscillateur de puissance HF (10) servant à exciter le circuit de sortie (35) étant configuré dans un deuxième mode de fonctionnement pour délivrer une deuxième fréquence d'excitation, différente de la première fréquence d'excitation et qui ne correspond pas à la fréquence de résonance du circuit de sortie (35),
**caractérisé en ce que**
l'oscillateur de puissance HF (10) comporte des éléments de commutation, en particulier des transistors (50, 55, 60, 65), qui commandent le signe de la tension d'excitation et par lesquels l'oscillateur de puissance HF (10) excite le circuit de sortie (35) en fonction du signe du courant d'excitation dans un premier mode de fonctionnement en rétroaction et qui établissent la deuxième fréquence d'oscillation dans un deuxième mode de fonctionnement forcé et
**en ce que** le générateur chirurgical de hautes fréquences comporte un appareil réglable (30) de commande de fréquence qui définit le signe du courant d'excitation et qui ouvre et/ou ferme les éléments de commutation qui établissent le signe de la tension d'excitation dans le premier mode de fonctionnement et qui établissent la deuxième fréquence d'excitation dans le deuxième mode de fonctionnement.

2. Générateur chirurgical de hautes fréquences selon la revendication 1, **caractérisé par** un dispositif asservi de commutation de mode de fonctionnement qui commute du premier mode de fonctionnement au deuxième mode de fonctionnement en vue de former une tension de sortie dotée d'harmoniques.

3. Procédé de conduite d'un générateur chirurgical de hautes fréquences doté d'un oscillateur de puissance HF (10) et d'un circuit de sortie (35), le procédé comportant les étapes suivantes :
- dans un premier mode de fonctionnement, excitation du circuit de sortie (35) par l'oscillateur de puissance HF (10) à une première fréquence d'excitation, le courant d'excitation qui s'écoule de l'oscillateur de puissance HF vers le circuit de sortie en vue de former la tension de sortie HF étant mis en oscillation et la première fréquence d'excitation correspondant à une fréquence de résonance du circuit de sortie (35),
- dans un deuxième mode de fonctionnement, excitation du circuit de sortie (35) par l'oscillateur de puissance HF (10) à une deuxième fréquence d'excitation différente de la première fréquence d'excitation et ne correspondant pas à la fréquence de résonance du circuit de sortie (35) pour diminuer de façon accélérée la tension de sortie HF,
- mesure du signe du courant d'excitation et
- ouverture et/ou fermeture d'éléments de commutation, en particulier de transistors (50, 55, 60, 65), qui commandent le signe de la tension d'excitation en fonction du signe du courant d'excitation dans le premier mode de fonctionnement en rétroaction et qui établissent la deuxième fréquence d'excitation dans un deuxième mode de fonctionnement forcé.
